# EUROPEAN PATENT SPECIFICATION

(11) **EP 4 079 829 B1**
(45) Date of publication and mention of the grant of the patent: **01.11.2023**
(21) Application number: 22166781.9
(22) Date of filing: 05.04.2022
(51) Int. Cl.: C10L 3/08, C12M 1/107, C12M 1/00

(54) **SAFETY SYSTEM FOR OPERATIONS ON A DIGESTER IN THE PRESENCE OF BIOGAS**
SICHERHEITSSYSTEM FÜR ARBEITEN AN EINEM FERMENTER IN ANWESENHEIT VON BIOGAS.
SYSTÈME DE SÉCURITÉ POUR TRAVAUX À UN DIGESTEUR EN PRÉSENCE DU BIOGAZ

(30) Priority: 20.04.2021 IT 202100009929
(43) Date of publication of application: 26.10.2022
(73) Proprietor: Alvus S.r.l., 39100 Bolzano BZ (IT)
(72) Inventor: Erckert, Christof, 39100 Bolzano BZ (IT)
(74) Representative: Perani & Partners S.p.A.

(56) References cited:
- JP-A- S57 165 096
- US-A1- 2011 245 572

## Description

### Technical field

The present invention relates to a safety system for operating safely near a digester and in the presence of biogas. Preferably, the safety system may be coupled to the digester at a digester platform for biogas plants and/or at digester active means, such as sprinklers or monitoring devices. Specifically, the safety system may be coupled to any part of the digester where, due to the presence of biogas, operators are subject to high risks for their health, as any possible biogas leakage may cause fires and/or explosions.

### State of the art

It is known in the state of the art carrying out operations on a digester, for example by active means, paying attention to possible biogas leaks caused during such operations. In fact, in order to avoid fires and/or explosions, the active means are positioned in areas of the digester that are open to the outside environment so as to allow as much as possible the dispersion into the environment of possible leaked biogas. Indeed, methane, the main component of the biogas produced in digesters, is flammable when mixed with oxygen and air in a volume concentration of between 5% and 15%. Below 5% methane is not sufficient to fuel combustion and for these reasons operations are carried out in direct contact with the external environment. Document US 2011/245572 A1 discloses a safety system for using carbon dioxide separated from biogas to be guided through closed storage containers and/or fermentation residue containers for inertization of explosive gas concentrations there. Document JP S57 165096 A discloses forming a space inside a tank and inertizing this space with the use of stored carbon dioxide.

### Problem of the prior art

The known techniques used to carry out operations on the digester do not allow to reduce nor control any possible biogas leakage, thus not allowing operators to work in full safety. During the leakage of the biogas, even if it is dispersible in the environment, in the presence of an ignition (e.g. a spark), methane might cause an explosion and/or a flame that might harm the operator.

### Summary of the invention

Within this framework, the technical task underlying the present invention is to provide a safety system which overcomes the drawbacks of the prior art.

In particular, it is an object of the present invention to provide a safety system capable of improving the safety of operators near a digester in the presence of biogas by exploiting at least in part the biogas produced by the digester itself.

The specified technical task and the specified objects are substantially achieved by a safety system for operations on a digester in the presence of biogas as defined in the enclosed claims.

### Advantages of the invention

Advantageously, the safety system of the present invention allows an operator to operate on a digester in an inert environment.

Advantageously, the safety system of the present invention allows a portion of the digester to be isolated to render it inert to the methane present in the biogas.

Advantageously, the safety system of the present invention allows to recycle the carbon dioxide present in the biogas to render inert a chamber within which it is possible to safely operate on the digester.

### BRIEF DESCRIPTION OF THE DRAWINGS

Further features and advantages of the present invention will become clearer from the indicative, and therefore non-limiting, description of a preferred, but not exclusive, embodiment of a safety system for operations on a digester in the presence of biogas shown with reference to the appended figures wherein:

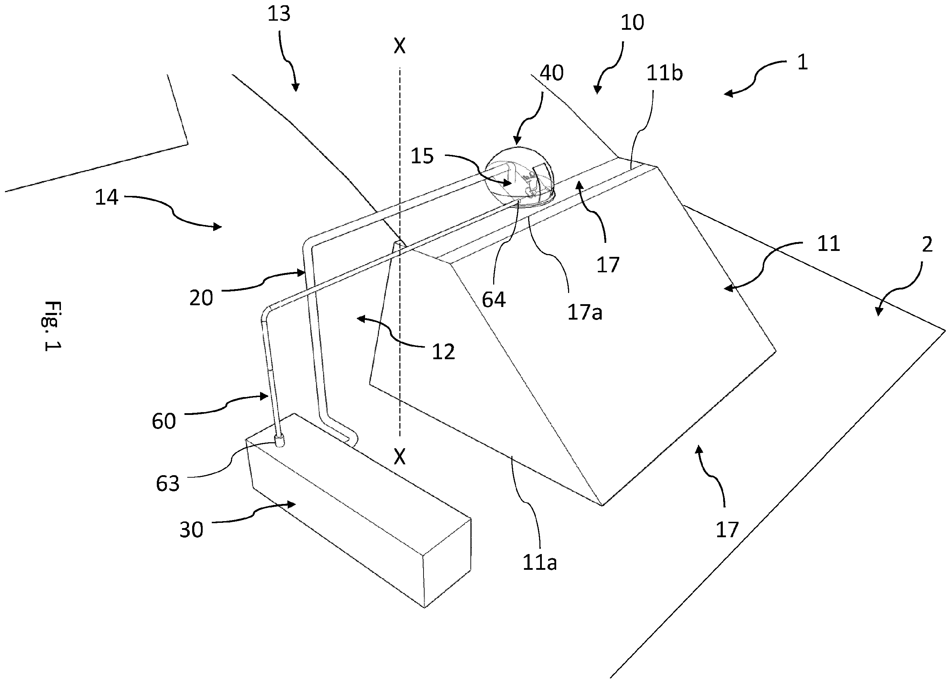

- Figure 1 shows a perspective section view of a safety system according to a preferred embodiment of the present invention with some parts removed to better highlight others;
- Figure 2 shows a perspective view of a detail of a safety system according to a preferred embodiment of the present invention

### DETAILED DESCRIPTION

Referring in particular to the appended figures, number 1 indicates the safety system for operations on a digester in the presence of biogas which is the subject of the present invention.

The system 1 comprises at least a digester 10 for biogas production arranged on a support ground 2. It should be noted that for the purposes of the present invention, the biogas is obtained by bacterial fermentation in anaerobiosis (absence of oxygen) of organic residues from: decomposing plant waste, zootechnical slurry or sewage sludge or agro-industrial waste or other organic waste.

The biogas thus produced is a mixture of methane and carbon dioxide and must be purified from carbon dioxide, as will be explained below, in order to deliver a mixture comprising mainly methane to the consumers. Specifically, the biogas mixture comprises about 50%-60% methane and the remaining 50%-40% carbon dioxide.

The digester 10 comprises a side wall 11, a cover 13 and active means 15.

Specifically, the side wall 11 extends from the support ground 2 along a height direction X-X transverse thereto. In detail, the side wall 11 rises transversely from the support ground 2. Preferably, the side wall 11 extends between a lower portion 11a associated with the support ground 2 and an opposite upper portion 11b spaced from the lower portion 11a along the height direction X-X perpendicular to the support ground 2.

The digester 10 also has one or more support surfaces 17 substantially parallel to the support ground 2 and associated with the side wall 11 or the support ground 2. Specifically, the support surfaces 17 comprise wall support surfaces 17a associated with the side wall 11 and base support surfaces associated with the support ground 2.

The wall support surfaces 17a are formed at the relative upper portion 11b of the side wall 11. The wall support surfaces 17a substantially parallel to the support ground 2 allow the movement of operators and/or working means at the cover 13 which, as clarified hereinafter, is coupled to the side wall 11 at the upper portion 11b. Regarding the base support surfaces, these are associated with the support ground 2 at the lower portion 1 1a of the side wall 11.

The side wall 11 defines a basin 12. The latter is configured to receive an amount of organic matter to generate biogas following bacterial fermentation in anaerobiosis. It should be noted that such a basin 12 may be used either to make tanks 14 for a digester 10, as shown below, and for storage tanks configured to receive processed organic matter exiting the digesters or raw organic matter before being inserted into a digester.

Preferably, the basin 12 comprises a base delimited by the side wall 12 and formed at the support ground 2. More preferably, the digester 10 comprises a digester base associated with the support ground 2 and substantially parallel thereto from which the side wall 11 delimiting the base of the basin 12 and defining the associated basin 12 extends.

The cover 13 is associated with the side wall 11 and defines with the basin 12 the tank 14. Specifically, the cover 13 is mechanically fixed to the side wall 11 thus defining with the basin 12 the tank 14 whether it is a digester tank or a storage tank. Preferably, the cover 13 is fixed to the side wall 11 at the upper portion 11b.

The active means 15 are configured to act inside the tank 14. Specifically, the active means 15 are configured to place the inside of the tank 14 in communication with the outside environment so as to allow one or more operators to act on the biogas and/or biomass. Preferably, the active means 15 comprise one or more of an irrigation system, a monitoring and sensor system, a substrate recovery system, a gas recovery system or a stirring system, a mixer, a heating system, a desulphurisation system.

The digester 10 has one or more active portions 16 formed on the side wall 11 and/or the cover 13 and associated with the active means 15. It is worth noting that the active portions 16 are portions on the side wall 11 and on the cover 13 at which the active means 15 are positioned. It is worth noting that each digester may comprise a plurality of active portions 16 spaced apart from each other on the side wall 11 and/or the cover 13.

Preferably, the active portions 16 are associated with relative support surfaces 17 so as to define a working area for the operators allowing them to act on the active means 15. More preferably, each active portion 16 is associated with a wall support surface 17a in case the active means 15 act on the cover 14, while it is associated with a base support portion in case the active means 15 act on the side wall 11.

The system 1 comprises biogas collection means 20 in fluid communication with the tank 14. Such biogas collection means 20 are configured to extract the biogas produced inside the tank 14. Specifically, the cover 13 is configured to collect the biogas generated by the organic matter contained inside the basin 11 and enclosed between the organic matter in the basin and the cover 14 itself. Preferably, the active means 15 comprise biogas collection means 20.

The system 1 comprises a separation unit 30 associated with the biogas collection means 20 and configured to separate methane from carbon dioxide included in the produced biogas.

According to the embodiment of the present invention shown in the figures, the system 1 comprises a cover element 40 removably fixed to an active portion 16 of the digester 10 and a support surface 17 proximate to the relative active portion 16. Such cover element 40 defines with the active portion 16 of the digester a safety chamber 50 in fluid communication with the separation unit 30 to receive the separated carbon dioxide and generate a methane inert atmosphere near the active portion 16 as well as the active means 15.

It is worth noting that according to an alternative embodiment which can be combined with the previous one, in case, for instance, no carbon dioxide is available from the separation unit 30, the safety system 1 comprises one or more containers, for example cylinders, which can be associated with the cover element 40 to provide carbon dioxide within the safety chamber 50 so as to safely carry out the activities on the active means 15 and without having to interrupt them.

Specifically, the safety chamber 50 is configured to at least partially isolate the active portion 16 from the external environment to generate, by means of carbon dioxide, insufflated therein, the inert methane atmosphere. In other words, the safety chamber 50 is configured to delimit and isolate from the external environment an area near the active portion 16 to promote the creation of a methane inert atmosphere.

Advantageously, the safety chamber 40 allows to prevent possible explosions and flares by rendering the area close to the active portion 16, where the likelihood of biogas leakage is highest, inert.

Preferably, the cover element 40 is movable from one active portion 16 to another in order to define a relative safety chamber 50 should it be necessary to act on the active means 15 of one active portion 16 or another.

According to a preferred embodiment, the safety chamber 50 is configured to define in its inside in proximity to the active zone 16 a safety volume that can be filled with at least carbon dioxide to generate the methane inert atmosphere. It is worth noting that the safety volume close to the active zone 16 has the methane inert atmosphere comprising a carbon dioxide percentage equal to or higher than 75%, preferably equal to or higher than 65%, more preferably equal to or higher than 45%, most preferably equal to at least 30%. This makes it possible to reduce the percentage of oxygen in the chamber by rendering methane inert and/or maintaining a methane percentage equal to or lower than 5% inside the safety chamber 50.

Advantageously, the safety chamber 50 makes it possible to obtain an inert atmosphere by reducing the percentage of oxygen that can be mixed with the methane because, when the concentration by volume of methane is higher than 15%, it is the lack of oxygen that prevents combustion.

It is worth noting that according to the present invention the percentages are percentages by volume.

Preferably, the safety chamber 50 is configured to define therein the working area to allow operators to safely act on the active means 15 as they operate in an atmosphere inert to the methane that may leak from the digester near the active means 15.

According to a preferred embodiment, the safety chamber 50 has a fixed wall 51, a base 52 and a side wall 53. The fixed wall 51 is defined by the active portion 16 to which the cover element 40 is fixed and is formed on the digester 10. Specifically, the cover element 40, being coupled to the active position 16, delimits and defines the relative fixed wall 51.

Regarding the base 52 and the side wall 53, these are defined by the cover element 40. Specifically, the base 52 is associated with the support surface 17 proximate to the fixed wall 51. In other words, the base 52 rests on the support surface 17 either of the wall 17a or of the base. By contrast, the side wall 53 is connected to the base 52 and the fixed wall 51.

Preferably, the safety chamber 50 has a door to access 55 the safety chamber 50 formed on the side wall 53. More preferably, the access door 55 is facing the fixed wall 51. Such an access door 55 is configured to ease an operator entering the safety chamber 40.

According to a preferred embodiment, the cover element 40 has a base portion 41 that defines the base 52 and is configured to fit the relative support surface 17. The cover element 40 further comprises a side portion 42 extended from the base portion 41 and defining the side wall 53 of the safety chamber 50.

According to a preferred embodiment, the access door 55 is formed on the side portion 42 by means of a cut having two mutually separable flaps to allow entering into and exiting from the safety chamber 50. Preferably, they are provided first closing means to hold the flaps close to each other thus reducing alterations in the atmosphere within the safety chamber 50 when the cut is closed.

Alternatively, the access door 55 is formed on the side portion 42 by means of an inlet opening having an inlet perimeter. The latter is accessible by means of an inlet portion 46 configured to switch between a closed configuration in which it covers the inlet opening preventing an operator from entering into/exiting from the safety chamber 50 as well as the alteration of the atmosphere and an open configuration in which it clears the inlet opening allowing entering into/exiting from the safety chamber 50. In this case as well, they are provided second closing means to hold the portion of the inlet portion 46 close to the inlet perimeter thereby reducing alterations to the atmosphere within the safety chamber 50 in the closed configuration.

For example, the first and second closing means may be zips and/or Velcro^{®} fasteners.

It is worth noting that the cover element has a fixing portion 43 configured to fix the base portion 41 and the side portion 42 to the active portion 16 and define the fixed wall 51. Specifically, the fixing portion 43 is configured to couple with the active portion 16 by delimiting the fixed wall 51.

According to a preferred embodiment, the cover element 40 comprises one or more passage openings to allow the connections of the active means 15 from the active portion 16 to relative processing units, such as pumping systems or the separation unit 30. These passage openings may be sealed to adapt the safety chamber 50 to any active means 15 without altering the inert atmosphere therein. Preferably, seals may be associated with such passage openings so as to limit the exchange of fluid between the inside and outside of the safety chamber 50 upon the passage of a part of active means 15, such as a connecting conduit.

Preferably, the cover element 40 comprises a sheet made of a plastic material, for example, PVC-coated polyethylene. The sheet is shaped so as to be properly fixed to the active wall 16 and the support surface 17. Specifically, the sheet has a free perimeter and a central zone delimited by the free perimeter. The free perimeter defines the fixing portion 43 and is configured to couple with the active portion 16, while the central zone defines the base portion 41 and the side portion 42 which, by fixing the base portion 41 to the support surface 17, define the base 52 and the side wall 53 of the safety chamber 50 respectively.

According to a preferred embodiment, the system comprises connecting means 60 configured to place the separation unit 30 and the safety chamber 50 in fluid communication and to insufflate into the safety chamber 50 the carbon dioxide separated from the separation unit 30. It is thereby possible to recycle the carbon dioxide produced inside the digester and generate a methane inert atmosphere.

Advantageously, the use of the carbon dioxide produced reduces the costs required to render the active portions 16 inert.

According to a preferred embodiment, the connecting means 60 are also configured to place the safety chamber 50 in fluid communication with containers, such as cylinders, to provide carbon dioxide as an alternative and/or replacement for the carbon dioxide separated by the separation unit 30. Alternatively, the system 1 comprises further connecting means configured to place the containers in fluid communication with the safety chamber 50. In other words, the additional connecting means perform the same function of insufflating carbon dioxide as the combination of the connecting means 60 with the separation unit 30 and are connected and connectable to the cover element 40 in the same way as the connecting means 60.

Preferably, the connecting means 60 comprise a connecting conduit 61 extended between a first end 63 connected to the separation unit 30 (or to the carbon dioxide containers) and a second end 64 connected to the cover element 40. It is worth noting that the connecting duct 61 may be deformed and extended to reach any one of the active zones 16. Furthermore, such connecting conduit 61 is removably connected to the cover element 40 so as to ease moving the cover element 40 from one active portion 16 to another and/or mounting the safety chamber 50 and/or storing the cover element following operations on the active means 15. It is worth noting that according to a preferred embodiment, the cover element comprises a connecting device configured to couple with the duct 61 at the second end 64. Specifically, the connecting device comprises a hollow sleeve formed on the cover element and configured to allow the fluid connection between the two sides of the cover element. In other words, the sleeve is inserted into an opening formed on the cover element and fixed thereto. The conduit 61 is thereby connectable to the portion of the sleeve facing the external environment by known coupling types and insufflates carbon dioxide within the sleeve towards the active portion 16 as well as within the safety chamber 50.

According to a preferred embodiment, the system 1 comprises fixing means 70 and anchoring means 80 configured to removably fix the cover element 40 to the active portion 16 and the support surface 17 respectively.

The fixing means 70 comprising a first fixing element formed on the cover element 40 and a second fixing element formed on the active portion 16 and configured to couple with the first fixing element to fix the cover element 40 to the active portion 16.

Specifically, the second fixing means are positioned at an active portion 16 by circumscribing it, preferably by circumscribing on the side wall 11 and/or on the cover 14 the active means 15, so as to define a fixed wall 51 including the connection of the active means 15 to the digester associated with the relative active portion 16.

Preferably, the first fixing means are made at the fixing portion 43 of the cover element.

For example, the fixing means 70 may comprise zips and/or Velcro^{®} fasteners.

Instead, the anchoring means 80 comprising one or more anchoring elements 81 removably positioned on the cover element 40 inside or outside the safety chamber 60 and proximate to the support surface 17. Specifically, the anchoring elements 81 are configured to fix by gravity the cover element 40 to the support surface 17 whether it is a wall 17a or a base. In detail, the anchoring elements 81 are coupled to the base 52 of the safety chamber 50 as well as to the base portion 41 of the cover element 40 to fix it to the support surface 17. In other words, the anchoring elements 81 are configured to anchor the cover element 40 to the support surface 17.

According to a preferred embodiment, the safety system 1 comprises a platform 90 comprising a fixing portion 91 fixed to the side wall 11 and an operating portion 92 connected to the fixing portion 91 and comprising sealing means for the cover 13. It is worth noting that the active means 15 for operating inside the tank 14 are associated with the operating portion 92 of the platform 90 thus defining on the operating portion the relative active portion 16 and the wall support surface 17a on the first fixing portion 91. The cover element 40 thereby defines with the operating portion 92 the safety chamber 50. Specifically, the cover element 40 fixed to the operating portion 92 defines the fixed wall 51, fixed to the fixing portion 91 defines the base 52 and with the side portion 43 defines the side wall of the safety chamber 50.

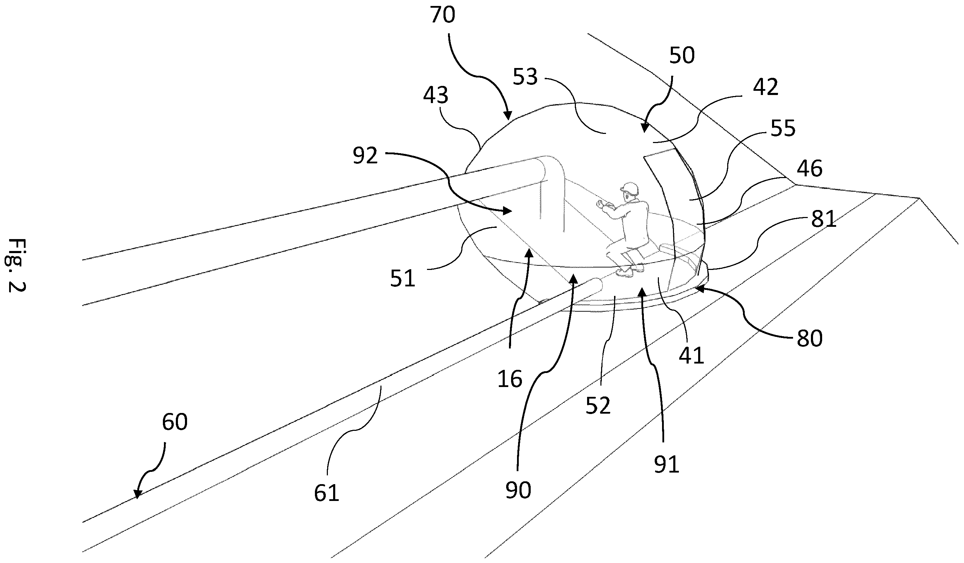

Referring in particular to Figure 2, the fixing portion 91 has a single profile counter-shaped to the wall support surface 17a of the side wall 11 to adhere entirely thereto. A counter-shaped profile of the fixing portion 91 means a profile that substantially reproduces the surface morphology of the wall support surface 17a, so that it can be fully adhered to it when the platform is installed in the digester.

Preferably, the operating portion 92 comprises coupling means (not shown in the accompanying figures) suitable for housing and supporting the active means 15.

According to a preferred aspect of the present invention, the operating portion 92 comprises a first plate suitable for being parallel to the bottom of the digester basin being used as well as the wall support surface 17a, and a second plate transverse to the first plate. Such first plate connects the fixing portion 91 and the second plate of the operating portion 92. Preferably, the second plate is orthogonal to the first plate. Even more preferably, the cover 13 of the digester is connected to the platform 90 at the second plate of the operating portion 92.

Advantageously, it is possible to install the active means 15 on the operating portion 92 to act on the surface section of the digester along a vertical direction orthogonal to the bottom of the digester basin. This makes it possible to move the biomass present inside the digester basin using an overhead irrigation system.

In an embodiment of the present invention, the operating portion 92 comprises side plates (not shown in the appended figures) connecting the first plate and the second plate to define the working area. Preferably, a user interacts in such working area with the active means 15 of the digester connected to the platform 90.

A further object of the present invention is a digester safety kit configured to enable the implementation of the safety system described above. The digester safety kit comprises the cover element 40 removably fixable to the active portion 16 and the support surface 17 proximate to the relative active portion 16 for defining with the active portion 16 the safety chamber 50.

The safety kit further comprises connecting means 60 configured to place the separation unit 30 (or carbon dioxide containers) and the safety chamber 50 in fluid communication and to insufflate within the safety chamber 50 at least part of the separated carbon dioxide. Preferably, the safety kit includes the additional connecting means and carbon dioxide containers so as to supply carbon dioxide to the safety chamber 50 if the separation unit 30 does not have it.

The safety kit also comprises fixing means 70 and anchoring means 80 configured to removably fix the cover element 40 to the active portion 16 and the support surface 17 respectively.

## Claims

1. Safety system for operations on a digester in the presence of biogas (1), the system (1) comprising:
- at least one digester (10) for biogas production arranged on a support ground (2) comprising
- a side wall (11) extending from the support ground (2) along a direction transverse thereto and defining a basin (12),
- a cover (13) associated with the side wall (11) and defining with the basin (12) a tank (14), and
- active means (15) configured to act inside the tank (14);
- the at least one digester (10) having:
- one or more active portions (16) formed on the side wall (11) and/or the cover (13) and associated with the active means (15);
- one or more support surfaces (17) substantially parallel to the support ground (2) and associated with the side wall (11) or the support ground (2);
- biogas collection means (20) in fluid communication with the tank (14) of the at least one digester (10) and configured to extract the produced biogas comprising methane and carbon dioxide;
- a separation unit (30) associated with the biogas collection means (20) and configured to separate methane from carbon dioxide included in the produced biogas;
**characterised in that it comprises:**
- a cover element (40) removably fixed to an active portion (16) of the digester (10) and to a support surface (17) proximate to the relative active portion (16), the cover element (40) defining with the active portion (16) of the digester a safety chamber (50) in fluid communication with the separation unit (30) to receive the separated carbon dioxide and generate a methane inert atmosphere near the active portion (16).

2. The system (1) according to claim 1, wherein the safety chamber (50) is configured to define in its inside in proximity to the active zone (16) a safety volume fillable with at least carbon dioxide to generate the methane inert atmosphere.

3. The system (1) according to claim 1 or 2, wherein the safety chamber (50) has:
- a fixed wall (51) defined by the active portion (16) and formed on the digester (10);
- a base (52) and a side wall (53) defined by the cover element (40), the base (52) being associated with the support surface (17) proximate to the fixed wall (51) and the side wall (53) being connected to the base (52) and to the fixed wall (51);
- a door to access (55) the safety chamber (50) formed on the side wall (53).

4. The system (1) according to claim 3, wherein the cover element (40) has:
- a base portion (41) defining the base (52) and being configured to fit the support surface (17);
- a side portion (42) extending from the base portion (41) and defining the side wall (53);
- a fixing portion (43) configured to fix the base portion (41) and the side portion (42) to the active portion (16) and define the fixed wall (51).

5. The system (1) according to any one of claims 1 to 4, wherein the cover element (40) comprises a sheet made of a plastic material.

6. The system (1) according to any one of claims 1 to 5, wherein the system (1) comprises connecting means (60) configured to place the separation unit (30) and the safety chamber (50) in fluid communication and to insufflate within the safety chamber (50) at least part of the separated carbon dioxide.

7. The system (1) according to claim 6, wherein the connecting means (60) comprise a connecting conduit (61) extended between a first end (63) connected to the separation unit (30) and a second end (64) connected to the cover element (40).

8. The system (1) according to any one of claims 1 to 7, wherein the following are provided:
- fixing means (70) configured to removably fix the cover element (40) to the active portion (16), the fixing means (70) comprising a first fixing element formed on the cover element (40) and a second fixing element formed on the active portion (16) and configured to couple with the first fixing element to fix the cover element (40) to the active portion (16);
- anchoring means (80) configured to removably anchor the cover element (40) to the support surface (3), anchoring means (80) comprising one or more anchoring elements (81) removably positioned on the cover element (40) inside or outside the safety chamber (60) and in proximity to the support surface (3).

9. The system (1) according to any one of claims 1 to 8, wherein there is provided a platform (90) comprising a fixing portion (91) fixed to the side wall (11) and an operating portion (92) connected to the fixing portion (91) and comprising sealing means for the cover (13), the active means (16) being associated to the operating portion (92) of the platform (90).

10. Digester safety kit for making the safety system according to any one of claims 1 to 9, the digester safety kit comprising:
- a cover element (40) removably fixable to an active portion (16) and a support surface (17) proximate to the relative active portion (16), the cover element (40) being configured to define with the active portion (16) a safety chamber (50),
- connecting means (60) configured to place the separation unit (30) and the safety chamber (50) in fluid communication and to insufflate within the safety chamber (50) at least part of the separated carbon dioxide;
- fixing means (70) configured to removably fix the cover element (40) to the active portion (16); and
- anchoring means (80) configured to anchor the cover element (40) to the support surface (17).

## Patentansprüche

1. Sicherheitssystem für den Betrieb eines Fermenters in Anwesenheit von Biogas (1), wobei das System (1) Folgendes umfasst:
- mindestens einen Fermenter (10) für die Biogaserzeugung, der auf einem Auflageboden (2) angeordnet ist, umfassend
- eine Seitenwand (11), die sich vom Auflageboden (2) in einer Richtung quer dazu erstreckt und ein Becken (12) begrenzt,
- eine Abdeckung (13), die mit der Seitenwand (11) verbunden ist und das Becken (12) definiert
einen Tank (14) und
- aktive Mittel (15), die so konfiguriert sind, dass sie im Inneren des Tanks (14) wirken;
- wobei der mindestens eine Fermenter (10) aufweist:
- ein oder mehrere aktive Abschnitte (16), die an der Seitenwand (11) und/oder der Abdeckung (13) ausgebildet und mit den aktiven Mitteln (15) verbunden sind;
- eine oder mehrere Auflageflächen (17), die im Wesentlichen parallel zum Auflageboden verlaufen und mit der Seitenwand (11) oder dem Auflageboden (2) verbunden sind;
- Biogassammelmittel (20), die in Fluidverbindung mit dem Tank (14) des mindestens einen Fermenters (10) stehen und so konfiguriert sind, dass sie das erzeugte Biogas, das Methan und Kohlendioxid umfasst, extrahieren;
- eine Trenneinheit (30), die mit den Biogassammelmitteln (20) verbunden und so konfiguriert ist, dass sie Methan von dem im erzeugten Biogas enthaltenen Kohlendioxid trennt;
**dadurch gekennzeichnet, dass es Folgendes umfasst:**
- ein Abdeckelement (40), das abnehmbar an einem aktiven Abschnitt (16) des Fermenters (10) und an einer Auflagefläche (17) in der Nähe des entsprechenden aktiven Abschnitts (16) befestigt ist, wobei das Abdeckelement (40) mit dem aktiven Abschnitt (16) des Fermenters eine Sicherheitskammer (50) definiert, die in Fluidverbindung mit der Trenneinheit (30) steht, um das abgetrennte Kohlendioxid aufzunehmen und eine Methan-Inertatmosphäre in der Nähe des aktiven Abschnitts (16) zu erzeugen.

2. System (1) nach Anspruch 1, wobei die Sicherheitskammer (50) so konfiguriert ist, dass sie in ihrem Inneren in der Nähe der aktiven Zone (16) ein Sicherheitsvolumen definiert, das mindestens mit Kohlendioxid gefüllt werden kann, um die Methan-Inertatmosphäre zu erzeugen.

3. System (1) nach Anspruch 1 oder 2, wobei die Sicherheitskammer (50) aufweist:
- eine feste Wand (51), die durch den aktiven Abschnitt (16) definiert ist und am Fermenter (10) ausgebildet ist;
- eine Basis (52) und eine Seitenwand (53), die durch das Abdeckelement (40) definiert sind, wobei die Basis (52) mit der Auflagefläche (17) in der Nähe der festen Wand (51) verbunden ist und die Seitenwand (53) mit der Basis (52) und der festen Wand (51) verbunden ist;
- eine Tür (55) für den Zugang zu der Sicherheitskammer (50), die an der Seitenwand (53) ausgebildet ist.

4. System (1) nach Anspruch 3, wobei das Abdeckelement (40) aufweist:
- einen Basisabschnitt (41), der die Basis (52) definiert und so konfiguriert ist, dass er auf die Auflagefläche (17) passt;
- einen Seitenabschnitt (42), der sich von dem Basisabschnitt (41) aus erstreckt und die Seitenwand (53) definiert,
- einen Befestigungsabschnitt (43), der so konfiguriert ist, dass er den Basisabschnitt (41) und den Seitenabschnitt (42) an dem aktiven Abschnitt (16) befestigt und die feste Wand (51) definiert.

5. System (1) nach einem der Ansprüche 1 bis 4, wobei das Abdeckelement (40) eine Folie aus einem Kunststoffmaterial umfasst.

6. System (1) nach einem der Ansprüche 1 bis 5, wobei das System (1) Verbindungsmittel (60) umfasst, die so konfiguriert sind, dass sie die Trenneinheit (30) und die Sicherheitskammer (50) in Fluidverbindung bringen und mindestens einen Teil des abgetrennten Kohlendioxids in die Sicherheitskammer (50) insufflieren.

7. System (1) nach Anspruch 6, wobei die Verbindungsmittel (60) eine Verbindungsleitung (61) umfassen, die sich zwischen einem ersten Ende (63), das mit der Trenneinheit (30) verbunden ist, und einem zweiten Ende (64), das mit dem Abdeckelement (40) verbunden ist, erstreckt.

8. System (1) nach einem der Ansprüche 1 bis 7, wobei Folgendes vorgesehen ist:
- Befestigungsmittel (70), die so konfiguriert sind, dass sie das Abdeckelement (40) abnehmbar an dem aktiven Abschnitt (16) befestigen, wobei die Befestigungsmittel (70) ein erstes Befestigungselement, das an dem Abdeckelement (40) ausgebildet ist, und ein zweites Befestigungselement, das an dem aktiven Abschnitt (16) ausgebildet ist und so konfiguriert ist, dass es mit dem ersten Befestigungselement koppelt, um das Abdeckelement (40) an dem aktiven Abschnitt (16) zu befestigen, umfassen;
- Verankerungsmittel (80), die so konfiguriert sind, dass sie das Abdeckelement (40) abnehmbar an der Auflagefläche (3) verankern, wobei die Verankerungsmittel (80) ein oder mehrere Verankerungselemente (81) umfassen, die abnehmbar an dem Abdeckelement (40) innerhalb oder außerhalb der Sicherheitskammer (60) und in der Nähe der Auflagefläche (3) angeordnet sind.

9. System (1) nach einem der Ansprüche 1 bis 8, wobei eine Plattform (90) vorgesehen ist, die einen Befestigungsabschnitt (91) umfasst, der an der Seitenwand (11) befestigt ist, und einen Betätigungsabschnitt (92), der mit dem Befestigungsabschnitt (91) verbunden ist, und wobei sie Dichtungsmittel für die Abdeckung (13) umfasst, wobei die aktiven Mittel (16) dem Betätigungsabschnitt (92) der Plattform (90) zugeordnet sind.

10. Fermentersicherheitsausrüstung zur Herstellung des Sicherheitssystems nach einem der Ansprüche 1 bis 9, wobei die Fermentersicherheitsausrüstung umfasst:
- ein Abdeckelement (40), das abnehmbar an einem aktiven Abschnitt (16) befestigt werden kann, und eine Auflagefläche (17) in der Nähe des entsprechenden aktiven Abschnitts (16), wobei das Abdeckelement (40) so konfiguriert ist, dass es mit dem aktiven Abschnitt (16) eine Sicherheitskammer (50) definiert,
- Verbindungsmittel (60), die so konfiguriert sind, dass sie die Trenneinheit (30) und die Sicherheitskammer (50) in Fluidverbindung bringen und mindestens einen Teil des abgetrennten Kohlendioxids in die Sicherheitskammer (50) insufflieren;
- Befestigungsmittel (70), die so konfiguriert sind, dass sie das Abdeckelement (40) abnehmbar an dem aktiven Abschnitt (16) befestigen; und
- Verankerungsmittel (80), die so konfiguriert sind, dass sie das Abdeckelement (40) an der Auflagefläche (17) verankern.

## Revendications

1. Système de sécurité pour des travaux sur un digesteur en présence de biogaz (1), le système (1) comprenant :
- au moins un digesteur (10) pour la production de biogaz agencé sur un socle de support (2) comprenant
- une paroi latérale (11) s'étendant à partir du socle de support (2) le long d'une direction transversale à celui-ci et définissant une cuve (12),
- une enveloppe (13) associée à la paroi latérale (11) et définissant, avec la cuve (12), un réservoir (14), et
- des moyens actifs (15) configurés pour agir à l'intérieur du réservoir (14) ;
- l'au moins un digesteur (10) ayant :
- une ou plusieurs parties actives (16) formées sur la paroi latérale (11) et/ou l'enveloppe (13) et associées aux moyens actifs (15) ;
- une ou plusieurs surfaces de support (17) sensiblement parallèles au socle de support et associées à la paroi latérale (11) ou au socle de support (2) ;
- des moyens de collecte de biogaz (20) en communication fluidique avec le réservoir (14) de l'au moins un digesteur (10) et configurés pour extraire le biogaz produit comprenant du méthane et du dioxyde de carbone ;
- une unité de séparation (30) associée aux moyens de collecte de biogaz (20) et configurée pour séparer le méthane du dioxyde de carbone inclus dans le biogaz produit ;
**caractérisé en ce qu'il comprend** :
- un élément enveloppe (40) fixé de manière amovible à une partie active (16) du digesteur (10) et à une surface de support (17) à proximité de la partie active (16) respective, l'élément enveloppe (40) définissant, avec la partie active (16) du digesteur, une chambre de sécurité (50) en communication fluidique avec l'unité de séparation (30) pour recevoir le dioxyde de carbone séparé et générer une atmosphère inerte pour le méthane près de la partie active (16).

2. Système (1) selon la revendication 1, dans lequel la chambre de sécurité (50) est configurée pour définir dans son intérieur, à proximité de la zone active (16), un volume de sécurité apte à être rempli avec au moins du dioxyde de carbone pour générer l'atmosphère inerte pour le méthane.

3. Système (1) selon la revendication 1 ou 2, dans lequel la chambre de sécurité (50) possède :
- une paroi fixe (51) définie par la partie active (16) et formée sur le digesteur (10) ;
- une base (52) et une paroi latérale (53) définies par l'élément enveloppe (40), la base (52) étant associée à la surface de support (17) à proximité de la paroi fixe (51) et la paroi latérale (53) étant reliée à la base (52) et à la paroi fixe (51) ;
- une porte pour accéder (55) à la chambre de sécurité (50) formée sur la paroi latérale (53).

4. Système (1) selon la revendication 3, dans lequel l'élément enveloppe (40) possède :
- une partie base (41) définissant la base (52) et étant configurée pour s'adapter à la surface de support (17) ;
- une partie latérale (42) s'étendant à partir de la partie base (41) et définissant la paroi latérale (53) ;
- une partie de fixation (43) configurée pour fixer la partie base (41) et la partie latérale (42) à la partie active (16) et définir la paroi fixe (51).

5. Système (1) selon l'une quelconque des revendications 1 à 4, dans lequel l'élément enveloppe (40) comprend une feuille constituée d'une matière plastique.

6. Système (1) selon l'une quelconque des revendications 1 à 5, dans lequel le système (1) comprend des moyens de liaison (60) configurés pour mettre l'unité de séparation (30) et la chambre de sécurité (50) en communication fluidique et pour insuffler au sein de la chambre de sécurité (50) au moins une partie du dioxyde de carbone séparé.

7. Système (1) selon la revendication 6, dans lequel les moyens de liaison (60) comprennent un conduit de liaison (61) s'étendant entre une première extrémité (63) reliée à l'unité de séparation (30) et une seconde extrémité (64) reliée à l'élément enveloppe (40).

8. Système (1) selon l'une quelconque des revendications 1 à 7, dans lequel les éléments suivants sont fournis :
- des moyens de fixation (70) configurés pour fixer de manière amovible l'élément enveloppe (40) à la partie active (16), les moyens de fixation (70) comprenant un premier élément de fixation formé sur l'élément enveloppe (40) et un second élément de fixation formé sur la partie active (16) et configuré pour se coupler avec le premier élément de fixation pour fixer l'élément enveloppe (40) à la partie active (16) ;
- des moyens d'ancrage (80) configurés pour ancrer de manière amovible l'élément enveloppe (40) à la surface de support (3), les moyens d'ancrage (80) comprenant un ou plusieurs éléments d'ancrage (81) positionnés de manière amovible sur l'élément enveloppe (40) à l'intérieur ou à l'extérieur de la chambre de sécurité (60) et à proximité de la surface de support (3).

9. Système (1) selon l'une quelconque des revendications 1 à 8, dans lequel est prévue une plateforme (90) comprenant une partie de fixation (91) fixée à la paroi latérale (11) et une partie travaux (92) reliée à la partie de fixation (91) et comprenant des moyens d'étanchéité pour l'enveloppe (13), les moyens actifs (16) étant associés à la partie travaux (92) de la plateforme (90).

10. Kit de sécurité de digesteur pour la fabrication du système de sécurité selon l'une des revendications 1 à 9, le kit de sécurité de digesteur comprenant :
- un élément enveloppe (40) apte à être fixé de manière amovible à une partie active (16) et à une surface de support (17) à proximité de la partie active (16) respective, l'élément enveloppe (40) étant configuré pour définir, avec la partie active (16), une chambre de sécurité (50),
- des moyens de liaison (60) configurés pour mettre l'unité de séparation (30) et la chambre de sécurité (50) en communication fluidique et pour insuffler au sein de la chambre de sécurité (50) au moins une partie du dioxyde de carbone séparé ;
- des moyens de fixation (70) configurés pour fixer de manière amovible l'élément enveloppe (40) à la partie active (16) ; et
- des moyens d'ancrage (80) configurés pour ancrer l'élément enveloppe (40) à la surface de support (17).
